# DEMANDE DE BREVET EUROPEEN

(11) **EP 0 875 234 A1**
(43) Date de publication de la demande: **04.11.1998**
(21) Numéro de dépôt: 97400954.0
(22) Date de dépôt: 28.04.1997
(51) Int. Cl.: A61K 7/025, A45D 40/26, A45D 40/06, A45D 34/04

(54) **Composition et produit de maquillage des lèvres à base de polymère filmogène**

(71) Demandeur: L'OREAL, 75008 Paris (FR)
(72) Inventeur: de la Poterie, Valérie, 77820 LE CHATELET EN BRIE (FR); Gueret, Jean-Louis, 75018 Paris (FR)
(74) Mandataire: Lhoste, Catherine

(57) **Abrégé**

L'invention se rapporte à une composition de maquillage des lèvres commercialement viable, comprenant une quantité suffisante d'un système polymérique qui comprend au moins une dispersion de particules de polymère filmogène, ledit système permettant l'obtention d'un film capable de suivre le mouvement desdites lèvres et/ou sans transfert. Elle se rapporte aussi à un produit de maquillage commercialement viable comprenant un réservoir (2), contenant cette composition, et un moyen amovible de fermeture dudit récipient. Cette composition est en particulier fluide, ce qui nécessite l'emploi d'un organe d'application (4) par exemple du type feutre, et éventuellement d'un essoreur (6)

## Description

La présente invention a trait à une composition notamment cosmétique susceptible d'être appliquée sur les lèvres du visage. Ladite composition comprend en particulier une dispersion de particules de polymère filmogène et peut être utilisée en tant que produit de maquillage. Elle a aussi trait à un produit de maquillage contenant cette composition conditionnée de façon à être commercialisée.

Les compositions à appliquer sur les lèvres, telles que les rouge-à-lèvres classiques, se présentent généralement sous forme de stick, de pâte souple ou de pâte coulée, et comprennent des corps gras tels que des huiles, des composés pâteux et/ou des cires, et une phase particulaire généralement composée de charges et de pigments.

Ces compositions présentent toutefois l'inconvénient de transférer. On entend par là que la composition est susceptible de se déposer, au moins en partie, sur certains supports avec lesquels elle est mise en contact, tels que, par exemple, un verre, une tasse, un vêtement ou la peau. En se déposant, ladite composition laisse une trace sur ledit support. Il s'ensuit donc une persistance médiocre de la composition sur les lèvres et la nécessité de renouveler régulièrement son application.

Un autre inconvénient des compositions de l'art antérieur réside dans le problème de migration. On a en effet constaté que certaines compositions avaient tendance à se propager à l'intérieur des ridules et/ou des rides qui entourent les lèvres, engendrant un effet inesthétique que la consommatrice souhaite bien évidemment éviter.

On connaît des compositions de maquillage dites "sans transfert", susceptibles de pallier les inconvénients ci-dessus mentionnés elles comprennent généralement, parmi les corps gras qui les composent, des huiles volatiles, notamment des huiles de silicone volatiles et/ou des huiles hydrocarbonées volatiles. Toutefois, ces compositions conduisent à un maquillage très mat.

Depuis plusieurs années, de nombreux cosméticiens se sont intéressés aux compositions cosmétiques, notamment de rouge-à-lèvres ou de fond de teint "sans transfert". Ainsi, il a été envisagé des compositions de rouge-à-lèvres "sans transfert" contenant de 1 à 70 % en poids de résine liquide de silicone à motifs répétitifs silicates, de 10 à 98 % en poids d'une huile de silicone volatile et des charges pulvérulentes. Toutefois, le film obtenu sur les lèvres après évaporation de l'huile de silicone présente l'inconvénient de devenir inconfortable au cours du temps (sensation de dessèchement et de tiraillement).

On connaît également des rouge-à-lèvres "sans transfert" contenant une silicone volatile et une résine de silicone comportant une chaîne estérifiée pendante ayant au moins 12 atomes de carbone. Le film de rouge-à-lèvres présente notamment l'inconvénient de manquer de confort à l'application, en particulier d'être trop sec. Ainsi, d'une manière générale, l'association d'huiles volatiles avec certains composés siliconés permet d'obtenir un résultat "sans transfert" satisfaisant. Toutefois, les films obtenus après application de ces compositions et évaporation des volatils présentent néanmoins l'inconvénient d'être relativement mats, et conduisent ainsi à un maquillage peu brillant.

Il subsiste donc le besoin d'une composition cosmétique qui transfère peu ou pas du tout, c'est-à-dire d'une composition "sans transfert", et qui possède également de bonnes propriétés cosmétiques telles que la facilité d'application, le confort et un démaquillage aisé.

De plus, les consommatrices sont encore dans l'attente d'une composition à appliquer sur les lèvres qui soit sans transfert et puisse donner un film brillant sur les lèvres.

En outre, il n'existe pas sur le marché de produit cosmétique de maquillage des lèvres commercialement viable contenant une composition ne transférant peu ou pas et qui, en plus, peut être brillant.

Par commercialement viable, on entend par là, notamment, un produit qui peut être fabriqué industriellement, vendu dans les circuits de distribution usuels et utilisé par les consommatrices pendant plusieurs semaines, voire plusieurs mois.

La présente invention a pour but de proposer une composition qui permet d'obtenir un film de très bonne tenue, qui ne transfère pas et ne tache pas un support avec lequel il serait en contact, et qui ne migre pas au cours du temps, tout en permettant d'obtenir un film brillant sur les lèvres.

Ainsi, un objet de l'invention est une composition de maquillage des lèvres, commercialement viable, comprenant une quantité suffisante d'un système polymérique qui comprend au moins une dispersion de particules de polymère filmogène, dans un milieu, ledit système permettant l'obtention d'un film capable de suivre le mouvement desdites lèvres.

Un autre objet de l'invention est une composition de maquillage à appliquer sur les lèvres, commercialement viable, comprenant un système polymérique qui comprend au moins une dispersion de particules de polymère filmogène, ledit système étant présent en une quantité efficace pour permettre l'obtention d'un film sans transfert.

La composition selon l'invention permet l'obtention d'un film homogène, qui présente une texture légère et reste confortable à porter tout au long de la journée. Le film n'est pas du tout collant, tout en étant mou, souple, élastique et flexible sur la peau. Il adhère parfaitement sur les lèvres du visage ; il suit les mouvements des lèvres sur lesquelles il est déposé sans se craqueler et/ou se décoller et sans provoquer de sensation de tiraillement.
Le film est de très bonne tenue ; il ne transfère pas, ne migre pas, ne tache pas.

La composition selon l'invention trouve donc une application particulièrement intéressante dans le domaine du soin et/ou du maquillage des lèvres du visage, notamment en tant que rouge-à-lèvres ou "laque à lèvres".

De plus, on a constaté que le film obtenu pouvait être très brillant, ou plus ou moins mat ou satiné, selon la nature des constituants de la composition, d'où une gamme plus étendue de produits de maquillage, brillants, satinés ou mats, par rapport à l'art antérieur.

Parmi les polymères filmogènes utilisables dans le cadre de la présente invention, on peut citer les polymères synthétiques, de type polycondensat ou de type radicalaires, les polymères d'origine naturelle, et leurs mélanges.

On peut ainsi citer, parmi les polycondensats, les polyuréthannes anioniques, cationiques, non-ioniques ou amphotères, les polyuréthannes-acryliques, les polyuréthannes-polyvinylpirrolidones, les polyester-polyuréthannes, les polyéther-polyuréthannes, les polyurées, les polyurée/polyuréthannes, et leurs mélanges.
Le polyuréthanne peut être, par exemple, un copolymère polyuréthanne, polyurée/uréthanne, ou polyurée, aliphatique, cycloaliphatique ou aromatique, comportant seule ou en mélange :
- au moins une séquence d'origine polyester aliphatique et/ou cycloaliphatique et/ou aromatique, et/ou,
- au moins une séquence siliconée, substituée ou non, ramifiée ou non, par exemple polydiméthylsiloxane ou polyméthylphénylsiloxane, et/ou
- au moins une séquence comportant des groupes fluorés.

Les polyuréthannes tels que définis dans l'invention peuvent être également obtenus à partir de polyesters, ramifiés ou non, ou d'alkydes comportant des hydrogènes mobiles que l'on modifie par réaction avec un diisocyanate et un composé organique bifonctionnel (par exemple dihydro, diamino ou hydroxyamino), comportant en plus soit un groupement acide carboxylique ou carboxylate, soit un groupement acide sulfonique ou sulfonate, soit encore un groupement amine tertiaire neutralisable ou un groupement ammonium quaternaire.
Comme polyuréthanne utilisable selon l'invention on peut utiliser ceux commercialiser sous les dénominations NEOREZ R-981 par la société ZENECA, les SANCURE 875, SANCURE 2255, SANCURE 861 par la société SANNCOR.

Parmi les polycondensats, on peut également citer les polyesters, les polyesters amides, les polyesters à chaîne grasse, les polyamides, et les résines époxyesters.
Les polyesters peuvent être obtenus, de façon connue, par polycondensation de diacides aliphatiques ou aromatiques avec des diols aliphatiques ou aromatiques ou des polyols. Comme diacides aliphatiques, on peut utiliser l'acide succinique, l'acide glutarique, l'acide adipique, l'acide pimélique, l'acide subérique ou l'acide sébacique. Comme diacides aromatiques, on peut utiliser l'acide téréphtalique ou l'acide isophtalique, ou bien encore un dérivé tel que l'anhydride phtalique. Comme diols aliphatiques, on peut utiliser l'éthylène glycol, le propylène glycol, le diéthylène glycol, le néopentyl glycol, le cyclohexane diméthanol, le 4,4'-(1-méthylpropylidène)bisphénol. Comme polyols, on peut utiliser le glycérol, le pentaérythritol, le sorbitol, le triméthylol propane.
Les polyesters amides peuvent être obtenus de manière analogue aux polyesters, par polycondensation de diacides avec des diamines ou des amino alcools. Comme diamine, on peut utiliser l'éthylènediamine, l'hexaméthylènediamine, la méta- ou para-phénylènediamine. Comme aminoalcool, on peut utiliser la monoéthanolamine.
Comme monomère porteur de groupement anionique pouvant être utilisé lors de la polycondensation, on peut citer par exemple l'acide diméthylol propionique, l'acide trimellitique ou un dérivé tel que l'anhydride trimellitique, le sel de sodium de l'acide sulfo-3 pentanediol, le sel de sodium de l'acide 5-sulfo 1,3-benzène dicarboxylique.
Les polyesters à chaîne grasse peuvent être obtenus par l'utilisation de diols à chaîne grasse lors de la polycondensation.
Les résines époxyesters peuvent être obtenues par polycondensation d'acides gras avec un condensat aux extrémités α, ω - diépoxy.

Par polymère radicalaire, on entend un polymère obtenu par polymérisation de monomères à insaturation notamment éthylénique, chaque monomère étant susceptible de s'homopolymériser (à l'inverse des polycondensats).
Les polymères de type radicalaires peuvent être notamment des polymères, ou des copolymères, vinyliques, notamment des polymères acryliques.

Les polymères vinyliques peuvent résulter de la polymérisation de monomères à insaturation éthylénique ayant au moins un groupement acide et/ou des esters de ces monomères acides et/ou des amides de ces monomères acides.

On utilise de préférence des polymères radicalaires anioniques, c'est-à-dire des monomères ayant au moins un monomère à groupement acide.

Comme monomère porteur de groupement acide, on peut utiliser des acides carboxyliques insaturés α,β-éthyléniques tels que l'acide acrylique, l'acide méthacrylique, l'acide crotonique, l'acide maléique, l'acide itaconique. On utilise de préférence l'acide (méth)acrylique et l'acide crotonique, et plus préférentiellement l'acide (méth)acrylique.

Les esters de monomères acides sont avantageusement choisis parmi les esters de l'acide (méth)acrylique (encore appelé les (méth)acrylates), notamment des (méth)acrylates d'alkyle, en particulier d'alkyle en C1-C20, de préférence en C1-C8, des (méth)acrylates d'aryle, en particulier d'aryle en C6-C10, des (méth)acrylates d'hydroxyalkyle, en particulier d'hydroxyalkyle en C2-C6.
Parmi les (méth)acrylates d'alkyle, on peut citer le méthacrylate de méthyle, le méthacrylate d'éthyle, le méthacrylate de butyle, le méthacrylate d'isobutyle, le méthacrylate d'éthyl-2 hexyle, le méthacrylate de lauryle.
Parmi les (méth)acrylates d'hydroxyalkyle, on peut citer l'acrylate d'hydroxyéthyle, l'acrylate de 2-hydroxypropyle, le méthacrylate d'hydroxyéthyle, le méthacrylate de 2-hydroxypropyle.
Parmi les (méth)acrylates d'aryle, on peut citer l'acrylate de benzyle et l'acrylate de phényle.
Les esters de l'acide (méth)acrylique particulièrement préférés sont les (méth)acrylates d'alkyle.

Selon la présente invention, le groupement alkyle des esters peut être soit fluoré, soit perfluoré, c'est-à-dire qu'une partie ou la totalité des atomes d'hydrogène du groupement alkyle sont substitués par des atomes de fluor.

Comme amides des monomères acides, on peut par exemple citer les (méth)acrylamides, et notamment les N-alkyl (méth)acrylamides, en particulier d'alkyl en C2-C12. Parmi les N-alkyl (méth)acrylamides, on peut citer le N-éthyl acrylamide, le N-t-butyl acrylamide et le N-t-octyl acrylamide.

Les polymères vinyliques peuvent également résulter de l'homopolymérisation ou de la copolymérisation de monomères choisis parmi les esters vinyliques et les monomères styrèniques. En particulier, ces monomères peuvent être polymérisés avec des monomères acides et/ou leurs esters et/ou leurs amides, tels que ceux mentionnés précédemment.
Comme exemple d'esters vinyliques, on peut citer l'acétate de vinyle, le néodécanoate de vinyle, le pivalate de vinyle, le benzoate de vinyle et le t-butyl benzoate de vinyle.
Comme monomères styrèniques, on peut citer le styrène et l'alpha-méthyl styrène.

La liste des monomères donnée n'est pas limitative et il est possible d'utiliser tout monomère connu de l'homme du métier entrant dans les catégories de monomères acryliques et vinyliques (y compris les monomères modifiés par une chaîne siliconée).

Comme polymère acrylique utilisable selon l'invention, on peut citer ceux vendus sous les dénominations NEOCRYL XK-90, NEOCRYL A-1070, NEOCRYL BT-62, NEOCRYL A-1079, NEOCRYL A-523 par la société ZENECA, DOW LATEX 432 par la société DOW CHEMICAL.

Les polymères d'origine naturelle, éventuellement modifiés, peuvent être choisis parmi la résine shellac, la gomme de sandaraque, les dammars, les élémis, les copals, les dérivés cellulosiques, et leurs mélanges.

On peut encore citer les polymères résultant de la polymérisation radicalaire d'un ou plusieurs monomères radicalaires à l'intérieur et/ou partiellement en surface, de particules préexistantes d'au moins un polymère choisi dans le groupe constitué par les polyuréthannes, les polyurées, les polyesters, les polyesteramides et/ou les alkydes. Ces polymères sont généralement appelés polymères hybrides.

La dispersion comprenant un ou plusieurs polymères filmogènes peut être préparée par l'homme du métier sur base de ses connaissances générales.
La teneur en matière sèche desdites dispersions selon la présente invention peut être de l'ordre de 5-60% en poids, et de préférence 30-40%.
La composition peut comprendre 1-60% en poids, de préférence 5-40% en poids de matière sèche de polymères filmogènes.
La taille des particules de polymères en dispersion aqueuse peut être comprise entre 10-500 nm, et est de préférence comprise entre 20 et 150 nm.

Le milieu de la composition de l'invention est avantageusement un milieu aqueux contenant de l'eau et éventuellement des solvants miscibles à l'eau.

Lorsque le polymère filmogène ne permet pas d'obtenir seul un film ayant les caractéristiques mentionnées précédemment, il est possible d'ajouter un composé dont la fonction est de modifier les propriétés du polymère filmogène pour obtenir le système polymérique souhaité. On peut donc ajouter au polymère filmogène de la composition selon l'invention au moins un agent auxiliaire de filmification permettant d'obtenir un film ayant les caractéristiques telles que décrites précédemment. L'agent auxiliaire de filmification permet notamment d'obtenir un film mou, souple, qui suit bien le mouvement des lèvres.

Un tel agent auxiliaire de filmification peut être choisi parmi tous les composes connus de l'homme du métier comme étant susceptibles de remplir la fonction recherchée, et être notamment choisi parmi les agents plastifiants et les agents de coalescence. Ces derniers sont particulièrement utilisés lorsque le polymère selon l'invention se présente sous forme de dispersion aqueuse de particules de polymère filmogène. L'agent auxiliaire de filmification peut être soluble ou insoluble dans le milieu utilisé et peut éventuellement se présenter sous forme de dispersion notamment aqueuse.
En particulier, on peut citer, seuls ou en mélange, les plastifiants ou agents de coalescence usuels, tels que:
- les glycols et leurs dérivés tels que le diéthylène glycol éthyléther, le diéthylène glycol méthyléther, le diéthylène glycol butyléther ou encore le diéthylène glycol hexyléther, l'éthylène glycol éthyléther, l'éthylène glycol butyléther, l'éthyléne glycol hexyléther;
- les esters de glycérol,
- les dérivés de propylène glycol et en particulier le propylène glycol phényléther, le propylène glycol diacétate, le dipropylène glycol butyléther, le tripropylène glycol butyléther, le propylène glycol méthyléther, le dipropylène glycol éthyléther, le tripropylène glycol méthyléther et le diéthylène glycol méthyléther, le propylène glycol butyléther,
- des esters d'acides notamment carboxyliques, tels que des citrates, des phtalates, des adipates, des carbonates, des tartrates, des phosphates, des sébaçates,
- des dérivés oxyéthylénés tels que les huiles oxyéthylénées, notamment les huiles végétales telles que l'huile de ricin; les huiles de silicone,
- des polymères hydrosolubles ayant une température de transition vitreuse faible, inférieure à 25°C, de préférence inférieure à 15°C.

La quantité d'agent auxiliaire de filmification peut être choisie par l'homme du métier sur base de ses connaissances générales, de manière à obtenir un système polymérique conduisant à un film ayant les propriétés mécaniques souhaitées, tout en conservant à la composition des propriétés cosmétiquement acceptables.

Dans un mode de réalisation préféré, on peut utiliser un système polymérique tel qu'il permet l'obtention d'un film ayant au moins l'une des caractéristiques physico-chimiques suivantes :
- une élongation supérieure à environ 200 % de préférence supérieure à 300 % et mieux supérieure à 400, et/ou
- une dureté inférieure à environ 110, de préférence inférieure à 70, et préférentiellement inférieure à 55, la dureté étant de préférence supérieure à 1, et notamment supérieure à environ 5, et/ou
- un module de Young inférieur à environ 200 Mpa, de préférence inférieur à environ 100 Mpa, et préférentiellement inférieur à 80 Mpa, le module d'Young étant de préférence supérieur à 1 Mpa, et/ou
- un température de transition vitreuse (Tg) inférieure ou égale à 10° C et mieux 0° C.

Les méthodes de mesure d'élongation, de dureté et de module de Young (module d'élasticité) sont décrites avant les exemples.

La composition peut en outre comprendre au moins un colorant soluble dans le milieu et en particulier hydrosoluble et/ou au moins un pigment, utilisé de manière usuelle dans le domaine de la cosmétique et du maquillage des lèvres. Par pigments, il faut comprendre des particules blanches ou colorées, minérales ou organiques, insolubles dans le milieu, destinées à colorer et/ou opacifier la composition. Les pigments peuvent être présents dans la composition à raison de 0-20% en poids de la composition finale, et de préférence à raison de 1-5%. Ils peuvent être blancs ou colorés, minéraux et/ou organiques, de taille usuelle ou nanométrique. On peut citer, parmi les pigments et nanopigments minéraux, les oxydes de titane, de zirconium ou de cérium, ainsi que les oxydes de zinc, de fer ou de chrome, le bleu ferrique. Parmi les pigments organiques, on peut citer le noir de carbone, et les laques de baryum, strontium, calcium, aluminium. Parmi les colorants hydrosolubles, on peut citer les colorants usuels du domaine considéré tels que le sel disodique de ponceau, le sel disodique du vert d'alizarine, le jaune de quinoléine, le sel trisodique d'amarante, le sel disodique de tartrazine, le sel monosodique de rhodamine, le sel disodique de fuchsine, la xanthophylle, leurs mélanges.

Parmi les matières colorantes susceptibles d'être utilisées dans le domaine des produits à appliquer sur les lèvres, on peut citer les colorants références dans le Color Index CI 12085, CI 15850, CI 15985, CI 15985 laque Ba, CI 15985 laque Zr, CI 17200 , CI 19140 , CI 19140 laque Ba , CI 19140 laque Zr , CI 42053, CI 42090 , CI 45370, CI 45380, CI 45410, CI 45410 laques Ba, Sr, Zr, CI 47005, CI 61570 , CI 73360, CI 75120, CI 75130, CI 75170, CI 75470, CI 77163 , CI 77489 , CI 77491, CI 77492, CI 77499 , CI 77742 , CI 77891, CI 77947.

On peut également ajouter dans la composition selon l'invention tout additif usuel du domaine des compositions à appliquer sur les lèvres, tel que des agents épaississants, par exemple des argiles, des silices, des dérivés cellulosiques, des polymères synthétiques tel que polymère acrylique ou polymère associatif de type polyuréthanne; des gommes et notamment la gomme xanthane; des agents d'étalement; des dispersants; des conservateurs notamment hydrosolubles; des agents antimousses; des agents mouillants; des filtres UV; des parfums; des charges; des actifs cosmétiques ou pharmaceutiques; des hydratants; des vitamines et leurs dérivés; des matières biologiques et leurs dérivés.
Bien entendu l'homme du métier veillera à choisir ce ou ces éventuels additifs et/ou leur quantité, de manière telle que les propriétés avantageuses de la composition selon l'invention ne soient pas, ou substantiellement pas, altérées par l'adjonction envisagée.

Dans un mode de réalisation préféré, la composition selon l'invention peut comprendre :
- de 1 à 60 % en poids de matière sèche de polymère, de préférence de 5 à 40 % en poids, par rapport au poids total de la composition,
- de 1 à 8% en poids d'agent auxiliaire de filmification, de préférence de 1,5 à 5% en poids, par rapport au poids total de la composition,
- de 0 à 20 % en poids de pigments et/ou charges, de préférence de 1 à 5 % en poids
- des additifs cosmétiques
- le complément en milieu liquide notamment de l'eau.

La composition selon l'invention peut se présenter sous forme fluide, gélifiée, semi-solide, pâte souple, voire solide telle que de stick ou bâton.
Dans le cas d'une formule non solide, la composition selon l'invention peut présenter une viscosité allant de 0,05 Pa.s à 20 Pa.s (50 cPs à 20000 cPs), et notamment de 0,05 Pa.s à 10 Pa.s, mesurée à 25 °C à l'aide d'un appareil Brookfield, mobile 4 LVT.

Elle trouve en particulier une application en tant que produit de maquillage, notamment en tant que rouge à lèvres ou laque à lèvres, ou encore en tant que produit de soin des lèvres. La composition selon l'invention peut également être utilisée comme "overcoat", comme produit de base de maquillage des lèvres.

L'invention a donc également pour objet un procédé pour maquiller les lèvres du visage, dans lequel on utilise une composition telle que celles définies ci-dessus.

L'invention a aussi pour objet une laque à lèvres comprenant une quantité suffisante d'un système polymérique comprenant au moins une dispersion de polymère filmogène, dans un milieu, le système étant apte à former un film suivant les mouvements des lèvres.
Elle a aussi pour objet une laque à lèvres comprenant une quantité suffisante d'un système polymérique comprenant au moins une dispersion de polymère filmogène, dans un milieu, le système étant apte à former sur les lèvres un film sans transfert.

Un autre objet de l'invention est un produit de maquillage des lèvres comprenant d'une part la composition cosmétique à appliquer sur les lèvres du visage telle que décrite ci-dessus, et d'autre part un dispositif de conditionnement et d'application de ladite composition.

Le dispositif de conditionnement et d'application peut être choisi et fabriqué par l'homme du métier sur la base de ses connaissances générales, et adapté selon la nature de la composition à conditionner. En effet, le type de dispositif à employer peut être notamment lié à la consistance de la composition, en particulier à sa viscosité; il peut également dépendre de la nature des constituants présents dans la composition, la présence de composés volatils, par exemple.

D'une manière usuelle, le dispositif de conditionnement et d'application peut comprendre :
- un réservoir destiné à contenir la composition à appliquer, et
- un moyen amovible pour fermer, de préférence de manière étanche, ledit réservoir.
Ledit dispositif peut en outre comprendre un organe d'application de la composition cosmétique sur les lèvres, ledit organe d'application permettant le prélèvement de la composition et la restitution dudit produit prélevé lors de l'application du produit sur les lèvres.
Le dispositif peut également comprendre un organe d'essorage dudit organe d'application.
Cet organe d'application est de préférence solidaire des moyens de fermeture étanche du dispositif. De plus, l'organe d'essorage est de préférence solidaire du réservoir.

L'organe d'application peut être une plume, un feutre, un embout souple, un pinceau, une mousse, éventuellement floquée, un fritté.

Ainsi, lorsque la composition à appliquer sur les lèvres se présente sous la forme d'un produit de consistance liquide à pâteuse, le dispositif peut comporter, d'une manière connue, (1) un réservoir pour le produit à distribuer, (2) un embout d'application monté sur le réservoir et comportant une surface d'application munie d'au moins un orifice de distribution, et (3) des moyens aptes à provoquer l'expulsion du produit au travers de l'orifice de distribution, la surface d'application de l'embout comportant au moins une cavité au fond de laquelle l'orifice est situé. Un tel dispositif est notamment décrit dans les documents FR-A-2 727 608 et FR-A-2 727 909.

L'embout ou organe d'application peut être réalisé en une matière souple, par exemple en un matériau élastomérique souple ou semi-rigide qui peut être choisi dans le groupe des thermoplastiques, des élastomères thermoplastiques, des caoutchoucs naturels et synthétiques. L'embout d'application peut être en mousse alvéolée à cellules ouvertes ou fermées.

En particulier, l'embout d'application peut être réalisé en un matériau élastomérique choisi dans le groupe des élastomères de polyéthylène, de polyuréthanne, de polyester ; des polyester bloc amides ; des polyvinyles ; des ter-polymères d'éthyléne, de propylène et d'un diène (EPDM) ; des polymères de styrène-butadiène séquencés (SEBS-SIS).

peut être muni La surface d'application de l'embout d'application comporte avantageusement des aspérités régulièrement réparties sur toute ou partie de la surface de celle-ci.

De préférence, le revêtement de l'embout d'application est constitué d'un revêtement de flocage obtenu par des fibres naturelles ou synthétiques, telles que des fibres de coton, de polyamide, de polyester.

De préférence, l'embout d'application a une section choisie parmi les section circulaire, triangulaire et ovale. L'organe d'application peut être conformé sensiblement en forme d'ogive muni d'une surface d'application plane ou légèrement bombée vers l'intérieur ou vers l'extérieur, située en biais sur le flanc de l'ogive, équipé de l'orifice de distribution. Cet organe d'application peut comporter une multitude d'orifices de distribution de produit, par exemple en forme de grille.

Les moyens aptes à provoquer l'expulsion du produit comprennent notamment un piston apte à coulisser en translation à l'intérieur dudit réservoir, des moyens d'entraînement en translation traversant une fente longitudinale formée dans le réservoir, une embase raccordée audit réservoir, et des moyens d'entraînement en translation du piston. Le réservoir et l'embase sont réalisée avantageusement en une pièce et la fente est de préférence réalisée sur une partie seulement du réservoir. De plus, des moyens de maintien du piston dans le réservoir peuvent être prévus.

Dans un autre mode réalisation, ledit dispositif peut comporter (1) un réservoir pour le produit, muni d'un col et d'un organe d'application, (2) un élément de préhension dudit organe d'application monté sur ledit col, et (3) une tige ayant une première extrémité libre solidaire de l'organe d'application, destiné au prélèvement et à l'application du produit, et une seconde extrémité solidaire de l'élément de préhension.

L'organe d'application de cet autre mode peut être un feutre, un pinceau, un fritté, une mousse essentiellement floquée.

Ce dispositif peut comporter, en outre, un organe d'essorage annulaire fixé sur le col du réservoir et traversé par la tige, cet organe étant apte à essorer cette tige et/ou l'organe d'application. Avantageusement, cet organe d'essorage a la forme d'un doigt de gant pourvu d'un orifice de passage central qui peut être éventuellement floqué. Un tel dispositif est notamment décrit dans la demande de brevet français 96/02477 et dans le document FR-A-2 705 876.

Dans un autre mode de réalisation, le dispositif de conditionnement et d'application de la composition selon l'invention peut comprendre (1) un réservoir pour le produit, muni d'un col et (2) un bouchon pour fermer le col du réservoir, ledit bouchon étant équipé (3) d'un organe applicateur plongeant dans le réservoir lorsque le bouchon est en position de fermeture.

Avec un tel dispositif, on assure l'imprégnation de l'organe applicateur lorsque le réservoir est fermé, par exemple en agitant ou en retournant ledit dispositif de manière à favoriser le contact entre l'organe applicateur et le produit.

Le bouchon est ensuite retiré du réservoir pour permettre d'utiliser l'organe applicateur imprégné de produit, et d'appliquer ce produit à l'endroit souhaité. Un tel dispositif est notamment décrit dans le document EP-A-673 612.

Lorsque la composition selon l'invention est de viscosité plus importante, et se présente donc sous la forme d'un stick ou bâton de consistance sensiblement solide, l'ensemble de maquillage pour les lèvres peut comporter le produit en forme de raisin ou de bâton, (1) un support mobile, appelé cupule, recevant le produit à appliquer, (2) une enveloppe dans laquelle est montée la cupule sur laquelle vient se fixer (3) un capot et (4) un mécanisme permettant de déplacer en translation le raisin de façon à le loger dans l'enveloppe pendant la période de stockage, et de le sortir de son enveloppe lors de l'application du produit sur les lèvres. Un tel dispositif est notamment décrit dans le document FR-A-1 501 043.

On pourrait aussi utiliser le dispositif décrit dans le document FR-A-2 715 916.

L'invention est illustrée plus en détail dans les exemples suivants.

### A/ Mesure de l'élongation

L'élongation du film obtenu est mesurée selon la norme ASTM Standards, volume 06.01 D 2370-92 'Standard Test Method for Tensile Properties of Organic Coatings'.

### B/ Mesure de la dureté

La dureté du film est mesurée selon la norme ASTM D-43-66, ou la norme NF-T 30-016 (octobre 1981), à l'aide d'un pendule de Persoz.
Le film déposé sur le support doit avoir une épaisseur d'environ 300 microns avant séchage.

Après séchage pendant 24 heures, à 30°C et sous une humidité relative de 50%, on obtient un film ayant une épaisseur d'environ 100 microns; on mesure alors sa dureté à 30°C et 50% d'humidité relative.

### C/ Mesure du module de Young (ou module d'élasticité)

Le module de Young (module d'élasticité) est mesuré selon la norme ASTM Standards, volume 06.01 D 2370-92 'Standard Test Method for Tensile Properties of Organic Coatings'.
Le film déposé sur le support doit avoir une épaisseur d'environ 300 microns avant séchage. Après séchage pendant 7 jours à 21°C et sous une humidité relative de 50%, on obtient un film ayant une épaisseur d'environ 100 microns.
Les échantillons mesurés ont une largeur de 5 mm et une épaisseur de 100 microns. La distance entre les mors est de 25 mm. La vitesse de traction est de 1000 mm par minute.

La description qui suit se réfère aux figures annexées dans lesquelles :
- la figure 1 représente, en coupe longitudinale un produit de maquillage des lèvres conforme à l'invention
- la figure 2 est une vue en élévation d'un produit de maquillage des lèvres, selon un autre mode de réalisation de l'invention.

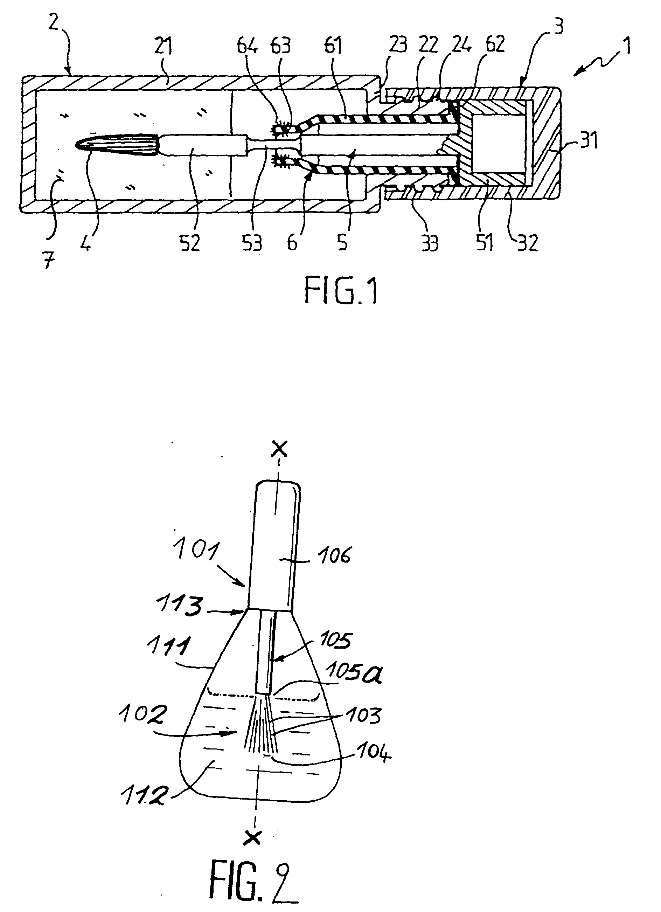

L'ensemble applicateur représenté sur la figure 1 est désigné dans son ensemble par la référence 1. Il est constitué par un flacon 2 renfermant la composition 7 à appliquer, un capuchon 3, formant organe de préhension, fixé sur le flacon 2, un organe d'application 4 porté par une tige 5 solidaire du capuchon 3. L'ensemble comprend également un organe d'essorage sous forme d'un essoreur annulaire 6, fixé sur le col du réservoir. L'organe d'essorage est de préférence sous forme d'un doigt de gant fixé au voisinage du col du réservoir. Dans le mode de réalisation illustré, l'applicateur 4 est sous forme d'une pointe feutre, laquelle peut être ou non recouverte d'un flocage.

Le flacon 2 est constitué d'un corps cylindrique 21 relié à un col 22 de plus faible diamètre par un épaulement 23. Le col 22 est muni extérieurement d'un filet 24. Le capuchon 3 comporte un fond plat 31 circulaire et une jupe cylindrique 32 à section circulaire, munie sur sa face intérieure d'un filet 33 susceptible de coopérer avec le filet 24 du col 22.

La pointe feutre est fixée sur la tige 5, par tout moyen approprié (collage, soudure, encliquetage, etc.). La tige 5 se termine par une jupe de fixation cylindrique 51 dont le diamètre externe est égal, au jeu nécessaire près, au diamètre interne de la jupe 32. La jupe 51 est montée à force dans le capuchon 3. La tige 5 est constituée par un élément cylindrique 52, muni d'un rétreint de plus faible diamètre 53 disposé de façon qu'en position de stockage, c'est à dire lorsque le capuchon 3 est vissé sur le flacon 2, ledit rétreint 53 se trouve au niveau de la lèvre de l'essoreur 6.

L'essoreur 6 représenté sur la figure 1 est du type à jupe. Il comporte un premier élément cylindrique 61, de diamètre extérieur égal, au jeu nécessaire près pour son montage, au diamètre interne du col. Ledit élément 61 est muni à une extrémité d'une collerette externe de retenue 62 qui vient reposer sur le bord libre du col 22. L'autre extrémité forme la lèvre 63 de l'essoreur 6. Cette lèvre peut être, au moins en partie, recouverte de fibres de flocage 64, ces fibres étant fixées sur le bord et sur une certaine hauteur des surfaces externe et interne de la lèvre 63.

En position de stockage, le capuchon 3 est vissé sur le col 22 du flacon 2. L'applicateur 4 est contenu dans le corps 21 du flacon 2, et plonge dans le produit à appliquer. Le rétreint de l'élément cylindrique 52 de la tige 5 est disposé au niveau de la lèvre 63 de l'essoreur 6.

Lorsque l'utilisatrice souhaite prélever du produit, elle dévisse le capuchon 3 et tire sur la tige 5 portant l'applicateur 4. La tige 52 passe dans la lèvre 63 de l'essoreur 6 où elle est en contact avec les fibres de flocage 64. La présence de fibres de flocage 64 entre la tige 52 et la lèvre 63 de l'essoreur 6 permet à l'air de pénétrer dans le flacon 2, évitant ainsi la formation d'une dépression. L'applicateur passe ensuite à travers la lèvre 63 de l'essoreur 6. La lèvre 63 élimine l'excès de produit chargé sur l'applicateur, lequel est prêt pour être utilisé. Après application du produit 7 sur les lèvres, l'utilisatrice rentre l'applicateur dans le flacon 2 et visse le capuchon 3. L'ensemble applicateur est prêt pour un nouvel usage.
La composition 7 est en particulier la laque à lèvres de l'exemple 2.

En se rapportant à la figure 2, on peut voir un ensemble de laque à lèvres conforme à l'invention comportant un pinceau (101) pour l'application de la laque (112) comprenant une touffe (102) de poils (103) fixée à une extrémité (105a) d'une tige (105) et orientée sensiblement suivant la direction axiale de la tige. Un manchon cylindrique (106) est solidaire de l'autre extrémité de la tige (105) opposée à la touffe (102) et engagé dans le manchon (106). Ce manchon cylindrique (106) sert d'organe de préhension du pinceau. Il sert également de bouchon destiné, par exemple, à être vissé de façon étanche sur le col (113) d'un flacon (111) contenant la laque à lèvres (112) telle que décrite à l'exemple 7. La touffe (102) est obtenue à partir d'un faisceau de poils (103) sensiblement parallèles replié en deux, environ à mi-longueur.

### Exemples de composition :

### Exemple 1 :

On prépare une formule de rouge à lèvres ayant la composition suivante :

| | |
|---|---|
| - dispersion aqueuse de polyuréthanne (SANCURE 861) | 38 g MA |
| - pigment | 2 g |
| - agent plastifiant (glycérine) | 1,25 g |
| - eau qsp | 100 g |

On obtient une composition facile à appliquer sur les lèvres qui donne un aspect satiné, qui ne transfère pas et ne coule pas.

Le SANCURE 861 a une élongation de 580 %, une dureté de 24, un module d'Young de 5,5 Mpa à 1000 mm/min et de 9,8 Mpa à 10 mm/min.

### Exemple 2 :

On prépare un rouge à lèvres ayant la composition suivante :

| | |
|---|---|
| - dispersion aqueuse de polyuréthanne (NEOREZ R-981) | 30,4 g MA |
| - pigment | 2 g |
| - agent plastifiant (glycérine) | 1,25 g |
| - eau qsp | 100 g |

On obtient une composition facile à appliquer sur les lèvres; le film obtenu est brillant; il ne transfère pas et ne migre pas dans les ridules ; il résiste bien et suit le mouvement des lèvres.

Le NEOREZ R-981 a une élongation de 330 %, une dureté de 104, un module d'Young de 168,1 Mpa à 1000 mm/min.

### Exemple 3 :

On prépare un rouge à lèvres ayant la composition suivante :

| | |
|---|---|
| - dispersion aqueuse de polyuréthanne (SANCURE 2255) | 46,55 g MA |
| - pigment | 2 g |
| - agent plastifiant (glycérine) | 1,25 g |
| - eau qsp | 100 g |

On obtient un film ayant les mêmes propriétés que dans l'exemple 2.

Le SANCURE 2255 a une élongation de 550 %, une dureté de 33,5, un module d'Young de 44,3 Mpa à 1000 mm/min et de 16,1 Mpa à 10 mm/min.

### Exemple 4 :

On prépare une composition fluide à appliquer sur les lèvres ayant la composition suivante :

| | |
|---|---|
| - dispersion aqueuse de polymère acrylique/styrène vendu sous la dénomination DOW LATEX 432 par la société DOW CHEMICAL | 25 g MA |
| - dispersion aqueuse de cire fluorée (MICRODISPERSION 411 de MICROPOWDERS) | 5 g MA |
| - pigment | 2 g |
| - agent plastifiant (glycérine) | 1,25 g |
| - eau qsp | 100 g |

On applique cette composition sur les lèvres et on obtient un film souple, qui ne craquèle pas et qui suit le mouvement des lèvres sans se décoller. En outre, le film ne transfère pas.

### Exemple 5 :

On prépare un rouge à lèvres fluide ayant la composition suivante :

| | |
|---|---|
| - dispersion aqueuse de polymère acrylique (NEOCRYL A-523 de la société ZENECA) | 20 g MA |
| - microdispersion de cire fluorée (MlCRODIPSERSION 411 de MICROPOWDERS) | 2,5 g MA |
| - glycérine (agent plastifiant) | 1,875 g |
| - épaississant | 0,5 g |
| - pigment | 3 g |
| - eau qsp | 100 g |

La composition s'étale facilement sur les lèvres et laisse un film souple, qui ne craquèle pas, confortable à porter, et qui ne transfère pas.

### Exemple 6 :

On prépare un rouge à lèvres fluide ayant la composition suivante :

| | |
|---|---|
| - dispersion aqueuse de polymère acrylique/styrène (NEOCRYL A-1052 de ZENECA) | 20 g MA |
| - acétyltributylcitrate | 2,5 g |
| - pigment | 2 g |
| - glycérine (agent plastifiant) | 1,25 g |
| - eau qsp | 100 g |

On obtient une composition facile à appliquer sur les lèvres. Le film obtenu ne trnansfère pas et présente une bonne tenue : il ne craquèle pas et suit le mouvement des lèvres.

### Exemple 7 :

On prépare un rouge à lèvres fluide ayant la composition suivante :

| | |
|---|---|
| - dispersion aqueuse de polymère acrylique/styrène (NEOCRYL A-1070 de ZENECA) | 42,75 g MA |
| - pigment | 2 g |
| - eau qsp | 100 g |

On obtient une composition facile à appliquer sur les lèvres. Le film obtenu ne transfère pas et présente une bonne tenue : il résiste bien et suit le mouvement des lèvres.

## Revendications

1. Composition de maquillage des lèvres, commercialement viable, comprenant une quantité suffisante d'un système polymérique qui comprend au moins une dispersion de particules de polymère filmogène, ledit système permettant l'obtention d'un film capable de suivre le mouvement desdites lèvres.

2. Composition de maquillage à appliquer sur les lèvres, commercialement viable, comprenant un système polymérique qui comprend au moins une dispersion de particules de polymère filmogène, ledit système étant présent en une quantité efficace pour permettre l'obtention d'un film sans transfert.

3. Composition selon la revendication 1 ou 2, caractérisée en ce que le système permet l'obtention d'un film ayant une dureté inférieure à 110 et mieux inférieure à 55.

4. Composition selon l'une des revendications précédentes, caractérisée en ce que le système permet l'obtention d'un film ayant une élongation supérieure à 200 % et mieux supérieure à 400 %.

5. Composition selon l'une des revendications précédentes, caractérisée en ce que le système permet l'obtention d'un film ayant un module Young inférieur à 200 MPa et mieux inférieur à 80 MPa.

6. Composition selon l'une des revendications précédentes, caractérisée en ce que le système polymèrique a une température de transition vitreuse inférieure à 10° Cet mieux inférieure à 0° C.

7. Composition selon l'une des revendications précédentes, dans laquelle le polymère filmogène est choisi parmi les polyuréthannes anioniques, cationiques, non ioniques ou amphotères ; les polyuréthannes-acryliques, les polyuréthannes-polyvinylpirrolidones, les polyester-polyuréthannes, les polyéther-polyuréthannes, les polyurée-polyuréthannes ; les polyesters, les polyesters amides, les polyesters à chaîne grasse, les polyamides, les résines époxyesters ; les polymères et/ou copolymères, acryliques et/ou vinyliques ; les copolymères acryliques/silicones ; les polymères d'origine naturelle, éventuellement modifiés ; les polymères résultant de la polymérisation radicalaire d'un ou plusieurs monomères radicalaires à l'intérieur et/ou partiellement en surface, de particules préexistantes d'au moins un polymère choisi dans le groupe constitué par les polyuréthannes, les polyurées, les polyesters, les polyesteramides et/ou les alkydes ; et leurs mélanges.

8. Composition selon l'une des revendications précédentes, dans laquelle la taille des particules de polymères en dispersion est comprise entre 10-500 nm, de préférence entre 20 et 150 nm.

9. Composition selon l'une des revendications précédentes, dans laquelle le système polymérique comprend en outre un agent auxiliaire de filmification.

10. Composition selon l'une des revendications précédentes caractérisée en ce qu'elle contient, en outre, au moins un colorant soluble dans le milieu et/ou au moins un pigment.

11. Composition selon l'une des revendications précédentes, dans laquelle le milieu est un milieu aqueux.

12. Produit de maquillage (1, 101) des lèvres, comprenant un réservoir (2, 111) contenant une composition (4, 112) de maquillage des lèvres et un moyen amovible de fermeture (3, 106) du récipient, caractérisé en ce que la composition est conforme à l'une des revendications précédentes.

13. Produit selon la revendication 12, caractérisé en ce qu'il comprend, en outre, un organe d'application (4, 103) de la composition sur les lèvres.

14. Produit selon la revendication 13, caractérisé en ce que l'organe d'application (4, 103) est porté par le moyen amovible de fermeture (3, 106).

15. Produit selon la revendication 13 ou 14, caractérisé en ce que l'organe d'application est une plume, un feutre (4), un embout souple, un pinceau (103), une mousse, éventuellement floqué, un fritté.

16. Produit selon l'une des revendications 12 ou 15, caractérisé en ce qu'il comprend, en outre, un organe d'essorage (6) de ladite composition.

17. Produit selon l'une des revendications 12 à 16, caractérisé en ce que la composition est fluide (112).

18. Produit selon l'une des revendications 12 ou 13, caractérisé en ce que la composition est pâteuse ou solide.

19. Produit selon la revendication 18, caractérisé en ce qu'il comprend, en outre, des moyens de déplacement en translation de ladite composition.

20. Laque à lèvres comprenant une quantité suffisante d'un système polymérique comprenant au moins une dispersion de polymère filmogène, dans un milieu, le système étant apte à former un film suivant les mouvements des lèvres.

21. Laque à lèvres comprenant une quantité suffisante d'un système polymérique comprenant au moins une dispersion de polymère filmogène, dans un milieu, le système étant apte à former sur les lèvres un film sans transfert.

22. Procédé pour maquiller les lèvres consistant à appliquer sur les lèvres une composition conforme à l'une des revendications 1 à 11 ou une laque à lèvres, conforme à l'une des revendications 21 ou 22.
